# DEMANDE DE BREVET EUROPEEN

(11) **EP 1 050 296 A1**
(43) Date de publication de la demande: **08.11.2000**
(21) Numéro de dépôt: 00400976.7
(22) Date de dépôt: 07.04.2000
(51) Int. Cl.: A61K 7/40, A61K 7/48

(54) **Composition renfermant au moins un composé bicyclique aromatique et au moins un filtre solaire lipophile, et ses utilisations**

(30) Priorité: 06.05.1999 FR 9905784
(71) Demandeur: L'OREAL, 75008 Paris (FR)
(72) Inventeur: Baldo, Francine, 92330 Sceaux (FR); Guiramand, Carole, 91310 Linas (FR)
(74) Mandataire: Renard, Emmanuelle

(57) **Abrégé**

La présente invention concerne une composition renfermant au moins un composé bicyclique aromatique de formule générale (I) : 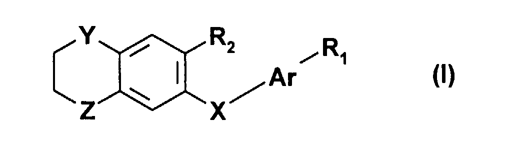 ou un sel ou analogue chiral d'un tel composé, caractérisée en ce qu'elle comprend en outre au moins un filtre solaire lipophile.

Le composé de formule (I) est de préférence l'acide 4-(3,5,5,8,8-pentaméthyl-5,6,7,8-tétrahydro-2-naphtylthio)benzoïque.

L'invention concerne également les utilisations de cette composition, ainsi qu'un procédé de solubilisation du composé de formule (I) ci-dessus au moyen d'au moins un filtre solaire lipophile.

## Description

La présente invention se rapporte à une composition renfermant au moins un composé bicyclique aromatique et au moins un filtre solaire lipophile, à des utilisations de ladite composition et à un procédé de solubilisation dudit composé par au moins un filtre solaire lipophile.

On connaît du brevet EP 679 630 des composés bicycliques aromatiques, ayant une activité du type rétinoïde, qui sont notamment utiles dans la prévention ou le traitement de différents troubles de kératinisation. Ces composés constituent donc des actifs cosmétiques de choix, en particulier dans la réparation ou la lutte contre le vieillissement chronologique ou actinique de la peau, par exemple comme anti-rides.

On a toutefois constaté que ces composés étaient difficiles à solubiliser dans des compositions cosmétiques. Ces actifs ont ainsi tendance à recristalliser ou à se dégrader. Il s'ensuit une perte d'efficacité plus ou moins importante des compositions qui les contiennent, selon le degré de recristallisation et/ou de dégradation, ce qui va à l'encontre de l'objectif recherché. En outre, cette recristallisation ou dégradation peut modifier la stabilité globale de ces compositions ainsi que leur aspect, ce qui peut détourner l'utilisateur de celles-ci.

Il subsiste donc le besoin de solubiliser ces composés dans un solubilisant physiologiquement acceptable.

Il est en outre connu de WO 97/31620 d'utiliser des filtres lipophiles tels que l'octocrylène et l'octyl méthoxycinnamate conjointement avec des composés à activité du type rétinoïde. Ces filtres lipophiles sont destinés à moduler l'irritation induite par les rétinoïdes en retardant et/ou modulant leur libération.

Les rétinoïdes auxquels il est essentiellement fait référence dans WO 97/31620 sont le rétinol, le rétinal et l'acide rétinoïque, qui sont effectivement des composés irritants. Au contraire, les composés bicycliques aromatiques mentionnés ci-dessus, qui présentent certes une activité de type rétinoïde analogue à celle du rétinol ou de l'acide rétinoïque, ont l'avantage par rapport aux rétinoïdes exemplifiés dans WO 97/31620 de ne pas être irritants.

Par suite, il n'était pas évident pour l'homme de l'art que l'association des composés bicycliques aromatiques selon l'invention avec des filtres solaires lipophiles présente un quelconque intérêt.

Or, la Demanderesse a maintenant constaté, de façon étonnante, que les filtres solaires lipophiles permettaient de solubiliser ces composés bicycliques aromatiques.

La présente invention a donc pour objet une composition renfermant au moins un composé bicyclique aromatique, caractérisée en ce qu'elle comprend en outre au moins un filtre solaire lipophile.

Elle a également pour objet un procédé de solubilisation d'un composé bicyclique aromatique, comprenant l'étape consistant à mélanger ledit composé bicylique à au moins un filtre solaire lipophile. Le mélange peut être effectué à froid, à température ambiante ou à chaud, par exemple à 80°C, généralement sous agitation.

Les composés bicycliques aromatiques selon l'invention ont précisément la formule générale (I) suivante : 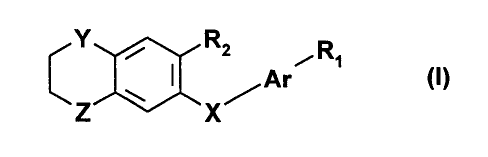 dans laquelle :
* R₁ représente un atome d'hydrogène, un radical -CH₃, un radical -CH₂-O-R₃, un radical -CH₂-O-CO-R₄, un radical -O-R₅, un radical -O-(CH₂)ₘ-(CO)ₙ-R₆, un radical -CO-R₇, un radical -CO-O-R₈, ou encore un radical -S(O)ₚ-R₉, les valeurs m, n et p ainsi que les différents radicaux R₃ à R₉ ayant la signification donnée ci-après,
* R₂ représente un atome d'hydrogène ou un atome d'halogène, un radical alkyle inférieur, un radical -NO₂, un radical -O-COR₄, un radical -OR₉ ou bien encore un radical 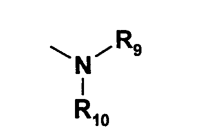 les radicaux R₄, R₉, et R₁₀ ayant la signification donnée ci-après,
* Ar représente un radical choisi parmi les radicaux de formules (a)-(e) suivantes : 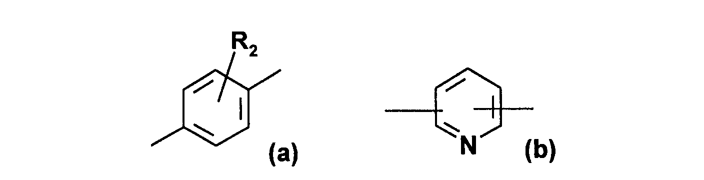 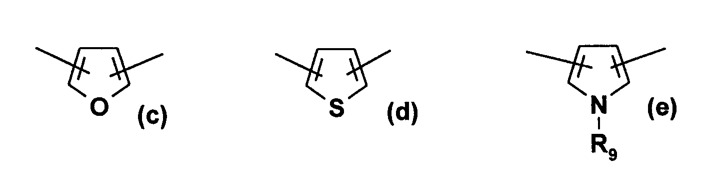 dans lesquelles R₂ a la signification ci-dessus, et R₉ celle donnée ci-après,
* X représente -O-, -S(O)ₜ- ou un radical -NR₉-, la valeur t et le radical R₉ ayant la signification donnée ci-après,
* Y et Z représentent -O-, -S(O)ₜ- ou encore un radical -CR₁₁R₁₂, la valeur t et les radicaux R₁₁ et R₁₂ ayant la signification donnée ci-après,
étant entendu que dans tout ce qui précède :
- m est un nombre entier égal à 1, 2 ou 3, n est un nombre entier égal à 0 ou 1, p est un nombre entier égal à 0, 1, 2 ou 3 et t est un nombre entier égal à 0, 1 ou 2,
- R_{3,} R_{5,} R_{9,} R_{10,} R_{11,} R₁₂ représentent, indépendamment les uns des autres, un atome d'hydrogène ou un radical alkyle inférieur,
- R₄ représente un radical alkyle inférieur,
- R₆ représente un radical alkyle inférieur ou un hétérocycle,
- R₇ représente un atome d'hydrogène, un radical alkyle inférieur ou un radical : 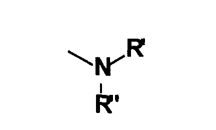 dans lequel R' et R", identiques ou différents, représentent un atome d'hydrogène, un radical alkyle inférieur, un radical mono ou polyhydroxyalkyle, un radical aryle éventuellement substitué ou un reste d'aminoacide ou de peptide ou de sucre ou encore, pris ensemble, forment un hétérocycle,
- R₈ représente un atome d'hydrogène, un radical alkyle linéaire ou ramifié ayant de 1 à 20 atomes de carbone, un radical alkényle, un radical mono ou polyhydroxyalkyle, un radical aryle ou aralkyle éventuellement substitué ou un reste de sucre ou un reste d'aminoacide ou de peptide,
- Y et Z ne peuvent représenter en même temps un atome d'oxygène ou un radical-S(O)ₜ-.

L'invention vise également les sels des composés de formule (I) ci-dessus dans les cas où le radical R₁ représente une fonction acide carboxylique ou une fonction acide sulfonique ou porte une fonction amine, ou encore lorsque le radical R₂ représente une fonction amine, ainsi que les analogues chiraux desdits composés. Lorsque les composés selon l'invention se présentent sous forme de sels, il s'agit de préférence de sels d'un métal alcalin ou alcalino-terreux, ou encore de zinc ou d'une amine organique.

Selon la présente invention, on entend par radical alkyle inférieur un radical ayant de 1 à 6 atomes de carbone, de préférence les radicaux méthyle, éthyle, isopropyle, butyle, tertiobutyle et hexyle.

Par radical alkyle linéaire ou ramifié ayant de 1 à 20 atomes de carbone, on entend notamment les radicaux méthyle, éthyle, propyle, 2-éthyl-hexyle, octyle, dodécyle, hexadécyle et octadécyle.

Par radical monohydroxyalkyle, on entend un radical ayant de préférence 2 ou 3 atomes de carbone, notamment un radical 2-hydroxyéthyle, 2-hydroxypropyle ou 3-hydroxypropyle.

Par radical polyhydroxyalkyle, on entend un radical contenant de préférence de 3 à 6 atomes de carbone et de 2 à 5 groupes hydroxyles tels que les radicaux 2,3-dihydroxypropyle, 2,3,4-trihydroxybutyle, 2,3,4,5-tétrahydroxypentyle ou le reste du pentaérythritol.

Par radical aryle, on entend de préférence un radical phényle éventuellement substitué par au moins un atome d'halogène, un hydroxyle ou une fonction nitro.

Par radical aralkyle, on entend de préférence le radical benzyle ou phénéthyle éventuellement substitué par au moins un atome d'halogène, un hydroxyle ou une fonction nitro.

Par radical alkényle, on entend un radical contenant de préférence de 2 à 5 atomes de carbone et présentant une ou plusieurs insaturations éthyléniques, tel que plus particulièrement le radical allyle.

Par reste de sucre, on entend un reste dérivant notamment de glucose, de galactose ou de mannose, ou bien encore de l'acide glucuronique.

Par reste d'aminoacide, on entend notamment un reste dérivant de la lysine, de la glycine ou de l'acide aspartique, et par reste de peptide on entend plus particulièrement un reste de dipeptide ou de tripeptide résultant de la combinaison d'acides aminés.

Par hétérocycle enfin, on entend de préférence un radical pipéridino, morpholino, pyrrolidino ou pipérazino, éventuellement substitué en position 4 par un radical alkyle en C₁-C₆ ou mono ou polyhydroxyalkyle tels que définis ci-dessus.

Lorsque le radical R₂ représente un atome d'halogène, celui-ci est de préférence un atome de fluor, de chlore ou de brome.

Selon la présente invention, les composés de formule (I) plus particulièrement préférés sont ceux pour lesquels l'une au moins, et de préférence toutes, les conditions ci-dessous sont respectées :
- R₁ est un radical -CO-R₇
- R₂ est un radical alkyl inférieur ou un radical -OR₉
- Ar représente un radical de formule (a)
- et X représente -O-, -S- ou -NR₉-.

Parmi les composés de formule (I) ci-dessus entrant dans le cadre de la présente invention, on peut notamment citer les suivants :
- l'acide 4-(5,6,7,8-tétrahydro-5,5,8,8-tétraméthyl-2-naphthyloxy)benzoïque
- l'acide 4-(5,6,7,8-tétrahydro-5,5,8,8-tétraméthyl-2-naphthylthio)benzoïque
- l'acide 4-(5,6,7,8-tétrahydro-5,5,8,8-tétraméthyl-2 naphthylsulfinyl) benzoïque
- l'acide 4-(5,6,7,8-tétrahydro-5,5,8,8-tétraméthyl-2-naphthylsulfonyl) benzoïque
- l'acide 4-(5,6,7,8-tétrahydro-5,5,8,8-tétraméthyl-2-naphthylamino) benzoïque
- l'acide 5-(5,6,7,8-tétrahydro-5,5,8,8-tétraméthyl-2-naphthylthio)-2-thiophene carboxylique
- l'acide 4-(3,5,5,8,8-pentaméthyl-5,6,7,8-tétrahydro-2-naphthyloxy) benzoïque
- l'acide 4-(3,5,5,8,8-pentaméthyl-5,6,7,8-tétrahydro-2-naphthylthio) benzoïque
- l'acide 4-(3-éthyl-5,5,8,8-tétramethyl-5,6,7,8-tétrahydro-2-naphthyloxy) benzoïque
- l'acide 4-(3-isopropyl-5,5,8,8-tétramethyl-5,6,7,8-tétrahydro-2-naphthyloxy) benzoïque
- la 4-(5,6,7,8-tétrahydro-5,5,8,8-tétraméthyl-2-naphthyloxy) acétophénone
- le 4-(5,6,7,8-tétrahydro-5,5,8,8-tétraméthyl-2-naphthyloxy) benzaldéhyde
- l'acide 4-(3-bromo-5,6,7,8-tétrahydro-5,5,8,8-tétramethyl-2-naphtyloxy) benzoïque
- l'acide 3-méthyl-4-(5,6,7,8-tétrahydro-5,5,8,8-tétramethyl-2-naphtylthio) benzoïque
- l'acide 3-méthyl-4-(3,5,5,8,8-pentaméthyl-5,6,7,8-tétrahydro-2-naphtylthio) benzoïque
- l'acide 3-méthyl-4-(3-éthyl-5,6,7,8-tétrahydro-5,5,8,8-tétramethyl-2-naphtyloxy) benzoïque
- l'acide 6-(3,5,5,8,8-pentaméthyl-5,6,7,8-tétrahydro-2-naphtylthio) nicotinique
- l'acide 2-hydroxy-4-(3,5,5,8,8-pentaméthyl-5,6,7,8-tétrahydrol-2-naphtylthio) benzoïque
- l'acide 2-chloro-4-(3,5,5,8,8-pentaméthyl-5,6,7,8-tétrahydro-2-naphtylthio) benzoïque
- l'acide 4-(3-éthyl-5,6,7,8-tétrahydro-5,5,8,8-tétramethyl-2-naphtylthio) benzoïque
- l'acide 4-(3-isopropyl-5,6,7,8-tétrahydro-5,5,8,8-tétramethyl-2-naphtylthio) benzoïque
- l'acide 4-(3-n-propyl-5,6,7,8-tétrahydro-5,5,8,8-tétramethyl-2-naphtylthio) benzoïque
- le 4-(3,5,5,8,8-pentaméthyl-5,6,7,8-tétrahydro-2-naphtylthio) benzèneméthanol
- le 4-(3,5,5,8,8-pentaméthyl-5,6,7,8-tétrahydro-2-naphtylthio) benzaldéhyde
- la N-éthyl-4-(3,5,5,8,8-pentaméthyl-5,6,7,8-tétrahydro-2-naphtylthio) benzamide
- la N-4-hydroxyphényl-4-(3,5,5,8,8-pentaméthyl-5,6,7,8-tétrahydro-2-naphtylthio) benzamide.

On préfère en particulier utiliser, dans la composition selon l'invention, l'acide 4-(3,5,5,8,8-pentaméthyl-5,6,7,8-tétrahydro-2-naphthylthio) benzoïque.

La composition selon la présente invention renferme, comme solubilisant des composés bicycliques aromatiques ci-dessus, au moins un filtre solaire lipophile.

Comme filtres solaires lipophiles convenant à une mise en oeuvre dans la présente invention, on peut notamment citer : les dérivés d'acide p-aminobenzoïque, tels que les esters, sels ou amides d'acide p-aminobenzoïque ; les dérivés d'acide salicylique tels que les esters ou sels d'acide salicylique ; les dérivés de benzophénone ; les dérivés de dibenzoylméthane ; les dérivés de diphénylacrylates ; les dérivés de benzofurannes ; les filtres UV polymères contenant un ou plusieurs résidus silico-organiques ; les esters d'acide cinnamique ; les dérivés de camphre ; les dérivés de trianilino-s-triazine ; l'acide phénylbenzimidazole sulfonique et ses sels ; l'acide urocanique ou son ester éthylique ; les benzotriazoles ; les dérivés d'hydroxyphényltriazine ; les bis-résorcinol-dialkylaminotriazine ; et leurs mélanges.

Le filtre solaire lipophile selon l'invention est de préférence choisi parmi : l'octyl salicylate ; la benzophénone-3 ; le butyl méthoxydibenzoylméthane ; l'octocrylène ; l'octyl méthoxy cinnamate et le composé de formule (II) suivante, ou 2-(2H-benzotriazole-2-yl)-4-méthyl-6-[2-méthyl-3-[1,3,3,3-tétraméthyl-1-[(triméthylsilyl)oxy] disiloxanyl]propynyl]phénol, décrit dans la demande de brevet EP-A-0 392 883 :

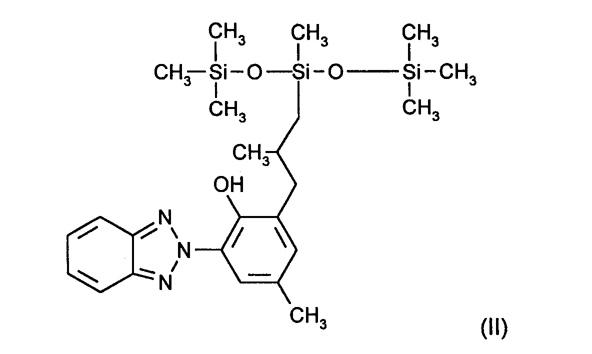

Le composé de formule (I) est présent dans la composition selon l'invention en une quantité efficace pour obtenir l'effet recherché, par exemple comprise entre 0,0001% et 10% en poids, de préférence entre 0,001 et 5% en poids, plus préférentiellement entre 0,01 et 1% en poids, par rapport à l'ensemble de la composition. En outre, le filtre solaire lipophile est présent dans la composition selon l'invention en une quantité suffisante pour solubiliser le composé de formule (I) et efficace pour conférer à la composition, seul ou en combinaison avec des filtres hydrophiles, le Facteur de Protection Solaire (SPF) voulu. La quantité de filtre lipophile est ainsi de préférence comprise entre 0,001% et 30% en poids par rapport à l'ensemble de la composition.

La composition selon l'invention peut se présenter sous toutes les formes galéniques normalement utilisées pour une application topique sur la peau, notamment sous forme d'une solution aqueuse, hydroalcoolique ou huileuse, d'une émulsion huile-dans-eau ou eau-dans-huile ou multiple, d'une émulsion siliconée, d'une microémulsion ou nanoémulsion, d'un gel aqueux ou huileux, d'un produit anhydre liquide, pâteux ou solide, d'une dispersion d'huile dans une phase aqueuse en présence de sphérules, ces sphérules pouvant être des nanoparticules polymériques telles que les nanosphères et les nanocapsules ou, mieux, des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse ou d'un gel. Elle peut éventuellement être appliquée sur la peau sous forme d'aérosol. Elle peut également se présenter sous forme solide, et par exemple sous forme de stick. Elle peut être utilisée comme produit de soin et/ou comme produit de maquillage de la peau, ou comme produit capillaire, par exemple comme shampooing ou après-shampooing.

De façon connue, la composition de l'invention peut contenir également les adjuvants habituels dans les domaines cosmétique et dermatologique, tels que les gélifiants hydrophiles ou lipophiles, les actifs hydrophiles ou lipophiles, les conservateurs, les antioxydants, les solvants, les parfums, les charges (telles que l'amidon, le talc ou le nylon), les pigments, les filtres hydrophiles, les absorbeurs d'odeur et les matières colorantes. Les quantités de ces différents adjuvants sont celles classiquement utilisées dans les domaines considérés, et par exemple de 0,01 à 20 % du poids total de la composition. Ces adjuvants, selon leur nature, peuvent être introduits dans la phase grasse, dans la phase aqueuse, dans les vésicules lipidiques et/ou dans les nanoparticules. Ces adjuvants, ainsi que leurs concentrations, doivent être tels qu'ils ne nuisent pas aux propriétés avantageuses du composé bicyclique aromatique selon l'invention.

Lorsque la composition selon l'invention est une émulsion, la proportion de la phase grasse peut aller de 5 à 80 % en poids, et de préférence de 5 à 50 % en poids par rapport au poids total de la composition. Les matières grasses, les émulsionnants et les coémulsionnants utilisés dans la composition sous forme d'émulsion sont choisis parmi ceux classiquement utilisés dans le domaine considéré. L'émulsionnant et le coémulsionnant sont de préférence présents, dans la composition, en une proportion allant de 0,3 à 30 % en poids, et de préférence de 0,5 à 20 % en poids par rapport au poids total de la composition.

Comme matières grasses utilisables dans l'invention, on peut utiliser les huiles et notamment les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser des alcools gras tels que l'alcool cétylique, des acides gras, des cires et des gommes et en particulier les gommes de silicone.

Il est particulièrement avantageux d'utiliser comme matières grasses au moins un alcool de Guerbet ou 2-alkyl alcan-1-ol, ou un ester d'un tel alcool, qui agissent comme co-solubilisants des composés bicycliques aromatiques. Des exemples de 2-alkyl alcan-1-ols convenant à une mise en oeuvre dans la présente invention sont notamment : le butyloctanol, l'hexyldécanol, l'octyldécanol, l'alcool isostéarylique, l'octyldodécanol, le décyltétradécanol, le undécylpentadécanol, le dodécylhexadécanol, le tétradécyloctadécanol, l'hexyldécyloctadécanol, le tétradécyleicosanol et le cétylarachidol. Comme esters desdits alcools, on peut citer : l'octanoate d'octyldodécyle ; le caprylate d'hexyldécyle ; le laurate d'hexyldécyle ; le palmitate d'hexyldécyle ; le stéarate d'hexyldécyle ; et l'octyldodécyle meadowfoamate, qui est un ester d'octyldodécanol et d'acides gras dérivés d'huile de germe de *Limnanthes Alba.*

Comme émulsionnants et coémulsionnants utilisables dans l'invention, il est particulièrement avantageux d'utiliser les esters d'acide gras et de polyol tels que le stéarate de PEG-100, le stéarate de PEG-50 et le stéarate de PEG-40 ; le stéarate de glycéryle ; le tristéarate de sorbitane, les stéarates de sorbitane oxyéthylénés disponibles sous les dénominations commerciales Tween® 20 ou Tween® 60, par exemple ; et leurs mélanges.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras et la silice hydrophobe.

Comme actifs, on peut utiliser notamment les dépigmentants, les émollients, les hydratants, les anti-séborrhéiques, les anti-acnéiques, les agents favorisant la repousse des cheveux, les agents kératolytiques et/ou desquamants, les agents anti-irritants, les agents apaisants et leurs mélanges. Avantageusement, dans la composition selon l'invention, les composés bicycliques aromatiques définis plus haut sont employés en combinaison avec d'autres composés à activité de type rétinoïde, avec des anti-radicaux libres, ou avec des α-hydroxy ou α-céto acides ou leurs dérivés. Par anti-radicaux libres, on entend par exemple l'α-tocophérol, la Super Oxyde Dismutate, l'Ubiquinol ou certains chélatants de métaux. Par α-hydroxy ou α-céto acides ou leurs dérivés, on entend par exemple les acides lactique, malique, citrique, glycolique, mandélique, tartrique, glycérique ou leurs sels, amides ou esters.

En cas d'incompatibilité, les actifs indiqués ci-dessus peuvent être incorporés dans des sphérules, notamment des vésicules ioniques ou non-ioniques et/ou des nanoparticules (nanocapsules et/ou nanosphères), de manière à les isoler les uns des autres dans la composition.

La composition selon l'invention trouve en particulier une application dans l'hygiène corporelle et capillaire et notamment pour le soin des peaux à tendance acnéique ou des peaux squameuses, pour lutter contre l'aspect gras de la peau ou des cheveux, pour lutter contre la chute des cheveux, dans la protection contre les aspects néfastes du soleil ou pour prévenir et/ou pour lutter contre le vieillissement actinique ou chronologique.

La présente invention concerne donc également l'utilisation de la composition mentionnée ci-dessus pour le traitement cosmétique de la peau, en particulier contre l'acné et/ou le vieillissement chronologique ou actinique. Elle concerne aussi l'utilisation de cette composition pour le traitement cosmétique du cuir chevelu, en particulier pour lutter contre l'aspect gras des cheveux et la chute des cheveux.

La présente invention concerne également l'utilisation de la composition mentionnée ci-dessus pour fabriquer une préparation destinée à lutter contre l'acné et/ou le vieillissement chronologique ou actinique de la peau. Elle concerne aussi l'utilisation de la composition mentionnée ci-dessus pour fabriquer une préparation destinée à lutter contre l'aspect gras des cheveux et la chute des cheveux.

Dans tous les cas, la composition selon l'invention et/ou la préparation obtenue à partir de celle-ci comprend une quantité efficace de composé bicyclique aromatique dans un milieu physiologiquement acceptable.

L'invention sera maintenant illustrée par les exemples non limitatifs suivants. Dans ces exemples, les quantités sont indiquées en pourcentage pondéral, sauf indication contraire.

### EXEMPLES

### Exemple 1

On a préparé l'émulsion huile-dans-eau ayant la composition suivante :

| *Phase A* | |
|---|---|
| Polyisobutène hydrogéné | 20 % |
| Octocrylène | 10 % |
| Alcool cétylique | 2,6 % |
| Stéarate de PEG-100 | 1,05 % |
| Stéarate de glycéryle | 1,05 % |
| Stéarate de sorbitan oxyéthyléné (20 OE) | 0,9 % |
| Composé bicyclique aromatique A* | 0,1 % |
| | |

| *Phase B* | |
|---|---|
| Glycérine | 3 % |
| Conservateurs | 0,46 % |
| Carbomer | 0,4 % |
| Gomme de xanthane | 0,1 % |
| Eau | 55,04 % |
| | |

| *Phase C* | |
|---|---|
| Triéthanolamine | 0,3 % |
| Eau | 5 % |

| | |
|---|---|
| *A : acide 4-(3,5,5,8,8-pentaméthyl-5,6,7,8-tétrahydro-2-naphthylthio) benzoïque | |

Les constituants de la phase A ont été mélangés, puis on a chauffé l'ensemble pendant une heure à 75-80°C. Les gélifiants (gomme de xanthane et carbomer) ont été dispersés dans le reste de la phase B sous agitation, puis l'ensemble a été chauffé à 75-80°C pendant une heure. La phase A a ensuite été introduite dans la phase B sous agitation pendant deux minutes à 75-85°C. La phase C a ensuite été ajoutée au mélange des phases A et B sous agitation, après arrêt du chauffage. On a enfin laissé refroidir l'émulsion obtenue jusqu'à 25°C.

On ne constate pas de formation de cristaux au moment de la préparation de l'émulsion, ni après 24 heures à 25°C (température ambiante). On peut donc considérer que la composition est stable pendant ces périodes de temps, aux températures indiquées.

### Exemple 2

On a préparé la même composition que celle décrite à l'Exemple 1, excepté qu'on a abaissé la quantité de polyisobutène hydrogéné à 12% et ajouté 8% d'hexyldécanol.

L'analyse macroscopique et microscopique de l'émulsion obtenue montre que celle-ci est stable après deux mois de conservation à 4°C, 25°C (température ambiante) et 45°C. En particulier, on ne constate pas de recristallisation du composé A après cette période de temps. En outre, la stabilité du composé A, telle que déterminée par HPLC, n'est pas affectée.

### Exemple 3

On a préparé la même composition que celle décrite à l'Exemple 1, excepté qu'on a substitué 7,5% en poids d'octyl méthoxycinnamate à l'octocrylène, la quantité d'eau de la phase B étant ajustée en conséquence.

On obtient les mêmes résultats de solubilité du composé A dans cette composition que dans celle de l'Exemple 1.

### Exemple 4

On a préparé la même composition que celle décrite à l'Exemple 3, excepté qu'on a abaissé la quantité de polyisobutène hydrogéné à 12% et ajouté 8% d'hexyldécanol.

L'analyse macroscopique et microscopique de l'émulsion obtenue montre que celle-ci est stable après deux mois de conservation à 4°C, 25°C (température ambiante) et 45°C. En particulier, on ne constate pas de recristallisation du composé A après cette période de temps. En outre, la stabilité du composé A, telle que déterminée par HPLC, n'est pas affectée.

### Exemple 5

On a préparé la même composition que celle décrite à l'Exemple 1, excepté qu'on a substitué à l'octocrylène 2% en poids d'une silicone benzotriazole, le 2-(2H-benzotriazole-2-yl)-4-méthyl-6-[2-méthyl-3-[1,3,3,3-tétraméthyl-1-[(triméthylsilyl)oxy] disiloxanyl]propynyl]phénol, la quantité d'eau de la phase B étant ajustée en conséquence.

On obtient les mêmes résultats de solubilité du composé A à 24 heures que dans l'Exemple 1.

### Exemple 6

On a préparé la même composition que celle décrite à l'Exemple 5, excepté qu'on a abaissé la quantité de polyisobutène hydrogéné à 12% et ajouté 8% d'hexyldécanol.

L'analyse macroscopique et microscopique de l'émulsion obtenue montre que celle-ci est stable après deux mois de conservation à 25°C (température ambiante) et 45°C. En particulier, on ne constate pas de recristallisation du composé A après cette période de temps, aux températures indiquées. En outre, la stabilité du composé A, telle que déterminée par HPLC, n'est pas affectée.

### Exemple 7

On a préparé la même composition que celle décrite à l'Exemple 1, excepté que la quantité d'octocrylène a été ramenée à 4% et que 1,5% en poids de butylméthoxy dibenzoylméthane et 0,3% en poids d'un séquestrant, le sel pentasodique d'acide éthylènediamine tétraméthylène phosphonique (à 33% dans l'eau), ont en outre été ajoutés à la composition.

On obtient les mêmes résultats de solubilité du composé A à 24 heures que dans l'Exemple 1.

### Exemple 8

On a préparé la même composition que celle décrite à l'Exemple 7, excepté qu'on a abaissé la quantité de polyisobutène hydrogéné à 12% et ajouté 8% d'hexyldécanol.

L'analyse macroscopique et microscopique de l'émulsion obtenue montre que celle-ci est stable après deux mois de conservation à 25°C (température ambiante) et 45°C, quoiqu'elle présente un aspect légèrement jaune. En particulier, on ne constate pas de recristallisation du composé A après cette période de temps, aux températures indiquées. En outre, la stabilité du composé A, telle que déterminée par HPLC, n'est pas affectée.

### Exemple 9 (comparatif)

On a préparé la même composition que celle décrite à l'Exemple 1, excepté que l'octocrylène a été substitué par 4,5% en poids d'acide téréphtalylidène-3,3'-camphosulfonique-10,10' à 33% dans l'eau, qui est un filtre solaire hydrophile décrit notamment dans le brevet français n° 82 10425 au nom de la Demanderesse. Le taux de triéthanolamine a en outre été porté à 1,15% en poids.

On constate que ce filtre hydrophile ne permet pas de solubiliser le composé A, même à 80°C sous agitation magnétique. Des cristaux sont visibles dès la formation de l'émulsion et à 24 heures.

### Exemple 10 (comparatif)

On a préparé la même composition que celle décrite à l'Exemple 1, excepté que l'octocrylène a été substitué par 2,5% en poids d'acide 2-phényl benzimidazole 5-sulfonique, qui est un filtre solaire hydrophile commercialisé par la société MERCK sous la dénomination commerciale EUSOLEX 232. Le taux de triéthanolamine a en outre été porté à 2,4% en poids.

On constate que ce filtre hydrophile ne permet pas de solubiliser le composé A, même à 80°C sous agitation magnétique. Des cristaux sont visibles dès la formation de l'émulsion et à 24 heures.

Il ressort de ce qui précède que, contrairement aux filtres hydrophiles, les filtres lipophiles permettent une bonne solubilisation du composé bicyclique aromatique selon l'invention. La solubilisation de ce composé peut encore être améliorée, sans affecter sa stabilité, par addition d'un alcool de Guerbet tel que l'hexyldécanol.

## Revendications

1. Composition comprenant au moins un composé bicyclique aromatique ayant la formule générale (I) suivante : 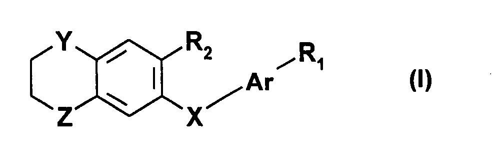 dans laquelle :
* R₁ représente un atome d'hydrogène, un radical -CH₃, un radical -CH₂-O-R₃, un radical -CH₂-O-CO-R₄, un radical -O-R₅, un radical -O-(CH₂)ₘ-(CO)ₙ-R₆, un radical -CO-R₇, un radical -CO-O-R₈, ou encore un radical -S(O)ₚ-R₉, les valeurs m, n et p ainsi que les différents radicaux R₃ à R₉ ayant la signification donnée ci-après,
* R₂ représente un atome d'hydrogène ou un atome d'halogène, un radical alkyle inférieur, un radical -NO₂, un radical -O-COR₄, un radical -OR₉ ou bien encore un radical 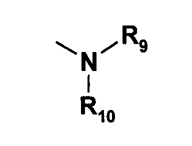 les radicaux R₄, R₉, et R₁₀ ayant la signification donnée ci-après,
* Ar représente un radical choisi parmi les radicaux de formules (a)-(e) suivantes : 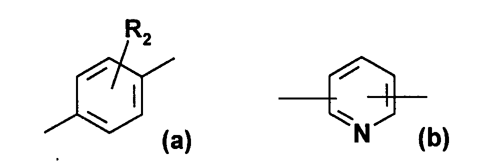 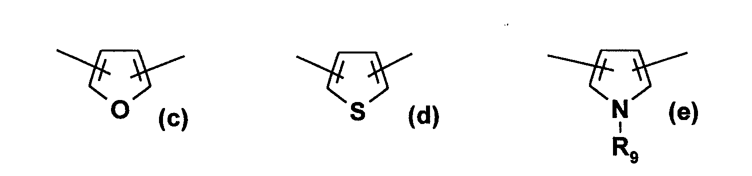 dans lesquelles R₂ a la signification ci-dessus, et R₉ celle donnée ci-après,
* X représente -O-, -S(O)ₜ- ou un radical -NR₉-, la valeur t et le radical R₉ ayant la signification donnée ci-après,
* Y et Z représentent -O-, -S(O)ₜ- ou encore un radical -CR₁₁R₁₂, la valeur t et les radicaux R₁₁ et R₁₂ ayant la signification donnée ci-après,
étant entendu que dans tout ce qui précède :
- m est un nombre entier égal à 1, 2 ou 3, n est un nombre entier égal à 0 ou 1, p est un nombre entier égal à 0, 1, 2 ou 3 et t est un nombre entier égal à 0, 1 ou 2,
- R_{3,} R_{5,} R_{9,} R_{10,} R_{11,} R₁₂ représentent, indépendamment les uns des autres, un atome d'hydrogène ou un radical alkyle inférieur,
- R₄ représente un radical alkyle inférieur,
- R₆ représente un radical alkyle inférieur ou un hétérocycle,
- R₇ représente un atome d'hydrogène, un radical alkyle inférieur ou un radical : 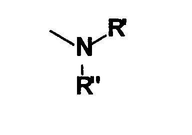 dans lequel R' et R", identiques ou différents, représentent un atome d'hydrogène, un radical alkyle inférieur, un radical mono ou polyhydroxyalkyle, un radical aryle éventuellement substitué ou un reste d'aminoacide ou de peptide ou de sucre ou encore, pris ensemble, forment un hétérocycle,
- R₈ représente un atome d'hydrogène, un radical alkyle linéaire ou ramifié ayant de 1 à 20 atomes de carbone, un radical alkényle, un radical mono ou polyhydroxyalkyle, un radical aryle ou aralkyle éventuellement substitué ou un reste de sucre ou un reste d'aminoacide ou de peptide,
- Y et Z ne peuvent représenter en même temps un atome d'oxygène ou un radical -S(O)ₜ-.
ou un sel ou analogue chiral d'un tel composé, caractérisée en ce qu'elle comprend en outre au moins un filtre solaire lipophile.

2. Composition selon la revendication 1, caractérisée en ce que ledit composé de formule (I) est tel que l'une au moins, et de préférence toutes, les conditions ci-dessous sont respectées :
- R₁ est un radical -CO-R₇
- R₂ est un radical alkyl inférieur ou un radical -OR₉
- Ar représente un radical de formule (a)
- et X représente -O-, -S- ou -NR₉-.

3. Composition selon la revendication 1 ou 2, caractérisée en ce que ledit composé de formule (I) est choisi parmi :
- l'acide 4-(5,6,7,8-tétrahydro-5,5,8,8-tétraméthyl-2-naphthyloxy) benzoïque
- l'acide 4-(5,6,7,8-tétrahydro-5,5,8,8-tétraméthyl-2-naphthylthio) benzoïque
- l'acide 4-(5,6,7,8-tétrahydro-5,5,8,8-tétraméthyl-2 naphthylsulfinyl) benzoïque
- l'acide 4-(5,6,7,8-tétrahydro-5,5,8,8-tétraméthyl-2-naphthylsulfonyl) benzoïque
- l'acide 4-(5,6,7,8-tétrahydro-5,5,8,8-tétraméthyl-2-naphthylamino) benzoïque
- l'acide 5-(5,6,7,8-tétrahydro-5,5,8,8-tétraméthyl-2-naphthylthio)-2-thiophène carboxylique
- l'acide 4-(3,5,5,8,8-pentaméthyl-5,6,7,8-tétrahydro-2-naphthyloxy) benzoïque
- l'acide 4-(3,5,5,8,8-pentaméthyl-5,6,7,8-tétrahydro-2-naphthylthio) benzoïque
- l'acide 4-(3-éthyl-5,5,8,8-tétramethyl-5,6,7,8-tétrahydro-2-naphthyloxy) benzoïque
- l'acide 4-(3-isopropyl-5,5,8,8-tétramethyl-5,6,7,8-tétrahydro-2-naphthyloxy) benzoïque
- la 4-(5,6,7,8-tétrahydro-5,5,8,8-tétraméthyl-2-naphthyloxy) acétophénone
- le 4-(5,6,7,8-tétrahydro-5,5,8,8-tétraméthyl-2-naphthyloxy) benzaldéhyde
- l'acide 4-(3-bromo-5,6,7,8-tétrahydro-5,5,8,8-tétramethyl-2-naphtyloxy) benzoïque
- l'acide 3-méthyl-4-(5,6,7,8-tétrahydro-5,5,8,8-tétramethyl-2-naphtylthio) benzoïque
- l'acide 3-méthyl-4-(3,5,5,8,8-pentaméthyl-5,6,7,8-tétrahydro-2-naphtylthio) benzoïque
- l'acide 3-méthyl-4-(3-éthyl-5,6,7,8-tétrahydro-5,5,8,8-tétramethyl-2-naphtyloxy) benzoïque
- l'acide 6-(3,5,5,8,8-pentaméthyl-5,6,7,8-tétrahydro-2-naphtylthio) nicotinique
- l'acide 2-hydroxy-4-(3,5,5,8,8-pentaméthyl-5,6,7,8-tétrahydrol-2-naphtylthio) benzoïque
- l'acide 2-chloro-4-(3,5,5,8,8-pentaméthyl-5,6,7,8-tétrahydro-2-naphtylthio) benzoïque
- l'acide 4-(3-éthyl-5,6,7,8-tétrahydro-5,5,8,8-tétramethyl-2-naphtylthio) benzoïque
- l'acide 4-(3-isopropyl-5,6,7,8-tétrahydro-5,5,8,8-tétramethyl-2-naphtylthio) benzoïque
- l'acide 4-(3-n-propyl-5,6,7,8-tétrahydro-5,5,8,8-tétramethyl-2-naphtylthio) benzoïque
- le 4-(3,5,5,8,8-pentaméthyl-5,6,7,8-tétrahydro-2-naphtylthio) benzéneméthanol
- le 4-(3,5,5,8,8-pentaméthyl-5,6,7,8-tétrahydro-2-naphtylthio) benzaldéhyde
- la N-éthyl-4-(3,5,5,8,8-pentaméthyl-5,6,7,8-tétrahydro-2-naphtylthio) benzamide
- la N-4-hydroxyphényl-4-(3,5,5,8,8-pentaméthyl-5,6,7,8-tétrahydro-2-naphtylthio) benzamide.

4. Composition selon la revendication 3, caractérisée en ce que ledit composé de formule (I) est l'acide 4-(3,5,5,8,8-pentaméthyl-5,6,7,8-tétrahydro-2-naphthylthio) benzoïque.

5. Composition selon l'une quelconque des revendications précédentes, caractérisée en ce qu'elle comprend de 0,0001% à 10% en poids de composé de formule (I).

6. Composition selon la revendication 5, caractérisée en ce qu'elle comprend de 0,001 à 5% en poids de composé de formule (I).

7. Composition selon la revendication 6, caractérisée en ce qu'elle comprend de 0,01 à 1% en poids de composé de formule (I).

8. Composition selon l'une quelconque des revendications précédentes, caractérisée en ce que ledit filtre solaire lipophile est choisi parmi :. les dérivés d'acide p-aminobenzoïque, tels que les esters, sels ou amides d'acide p-aminobenzoïque ; les dérivés d'acide salicylique tels que les esters ou sels d'acide salicylique ; les dérivés de benzophénone ; les dérivés de dibenzoylméthane ; les dérivés de diphénylacrylates ; les dérivés de benzofurannes ; les filtres UV polymères contenant un ou plusieurs résidus silico-organiques ; les esters d'acide cinnamique ; les dérivés de camphre ; les dérivés de trianilino-s-triazine ; l'acide phénylbenzimidazole sulfonique et ses sels ; l'acide urocanique ou son ester éthylique ; les benzotriazoles ; les dérivés d'hydroxyphényltriazine ; les bis-résorcinol-dialkylaminotriazine ; et leurs mélanges.

9. Composition selon la revendication 8, caractérisée en ce que ledit filtre solaire lipophile est choisi parmi : l'octyl salicylate ; la benzophénone-3 ; le butyl méthoxydibenzoylméthane ; l'octocrylène ; l'octyl méthoxycinnamate ; et le composé de formule (ll):

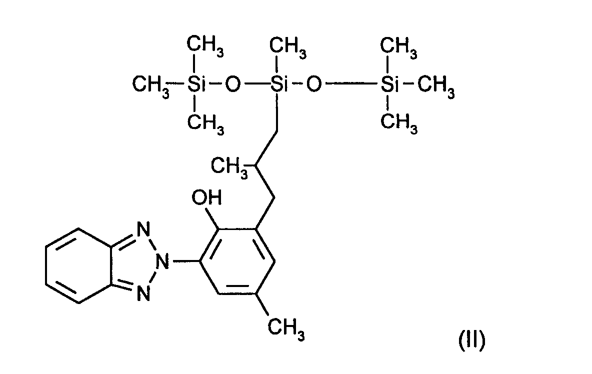

10. Composition selon l'une quelconque des revendications précédentes, caractérisée en ce qu'elle comprend de 0,001 à 30% en poids de filtre solaire lipophile.

11. Composition selon l'une quelconque des revendications précédentes, caractérisée en ce qu'elle renferme en outre au moins un co-solubilisant choisi parmi les 2-alkyl alcan-1-ols et leurs esters.

12. Composition selon la revendication 11, caractérisée en ce que le co-solubilisant est choisi parmi : le butyloctanol, l'hexyldécanol, l'octyldécanol, l'alcool isostéarylique, l'octyldodécanol, le décyltétradécanol, le undécylpentadécanol, le dodécylhexadécanol, le tétradécyloctadécanol, l'hexyldécyloctadécanol, le tétradécyleicosanol, le cétylarachidol, l'octanoate d'octyldodécyle, le caprylate d'hexyldécyle, le laurate d'hexyldécyle, le palmitate d'hexyldécyle, le stéarate d'hexyldécyle et l'octyldodécyle meadowfoamate.

13. Composition selon l'une quelconque des revendications précédentes, caractérisée en ce qu'elle comprend en outre au moins un émulsionnant choisi parmi : les esters d'acide gras et de polyol tels que le stéarate de PEG-100, le stéarate de PEG-50 et le stéarate de PEG-40 ; le stéarate de glycéryle ; le tristéarate de sorbitane, les stéarates de sorbitane oxyéthylénés ; et leurs mélanges.

14. Utilisation de la composition selon l'une quelconque des revendications précédentes pour le traitement cosmétique de la peau, en particulier contre l'acné et/ou le vieillissement chronologique ou actinique.

15. Utilisation de la composition selon l'une quelconque des revendications 1 à 13 pour le traitement cosmétique du cuir chevelu, en particulier pour lutter contre l'aspect gras des cheveux et la chute des cheveux.

16. Utilisation de la composition selon l'une quelconque des revendications 1 à 13 pour fabriquer une préparation destinée à lutter contre l'acné et/ou le vieillissement chronologique ou actinique de la peau.

17. Utilisation de la composition selon l'une quelconque des revendications 1 à 13 pour fabriquer une préparation destinée à lutter contre l'aspect gras des cheveux et la chute des cheveux.

18. Procédé de solubilisation d'un composé bicyclique aromatique ayant la formule générale (I) suivante : 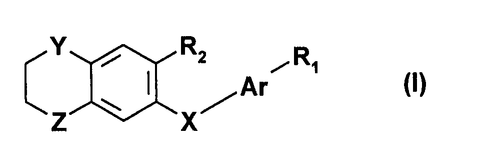 dans laquelle :
* R₁ représente un atome d'hydrogène, un radical -CH₃, un radical -CH₂-O-R₃, un radical -CH₂-O-CO-R₄, un radical -O-R₅, un radical -O-(CH₂)ₘ-(CO)ₙ-R₆, un radical -CO-R₇, un radical -CO-O-R₈, ou encore un radical -S(O)ₚ-R₉, les valeurs m, n et p ainsi que les différents radicaux R₃ à R₉ ayant la signification donnée ci-après,
* R₂ représente un atome d'hydrogène ou un atome d'halogène, un radical alkyle inférieur, un radical -NO₂, un radical -O-COR₄, un radical -OR₉ ou bien encore un radical 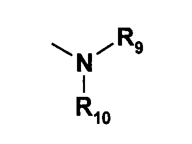 les radicaux R₄, R₉, et R₁₀ ayant la signification donnée ci-après,
* Ar représente un radical choisi parmi les radicaux de formules (a)-(e) suivantes : 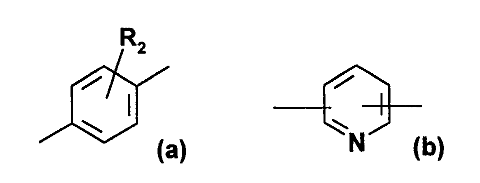 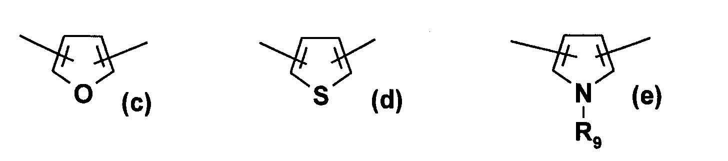 dans lesquelles R₂ a la signification ci-dessus, et R₉ celle donnée ci-après,
* X représente -O-, -S(O)ₜ- ou un radical -NR₉-, la valeur t et le radical R₉ ayant la signification donnée ci-après,
* Y et Z représentent -O-, -S(O)ₜ- ou encore un radical -CR₁₁R₁₂, la valeur t et les radicaux R₁₁ et R₁₂ ayant la signification donnée ci-après,
étant entendu que dans tout ce qui précède :
- m est un nombre entier égal à 1, 2 ou 3, n est un nombre entier égal à 0 ou 1, p est un nombre entier égal à 0, 1, 2 ou 3 et t est un nombre entier égal à 0, 1 ou 2,
- R_{3,} R_{5,} R_{9,} R_{10,} R_{11,} R₁₂ représentent, indépendamment les uns des autres, un atome d'hydrogène ou un radical alkyle inférieur,
- R₄ représente un radical alkyle inférieur,
- R₆ représente un radical alkyle inférieur ou un hétérocycle,
- R₇ représente un atome d'hydrogène, un radical alkyle inférieur ou un radical : 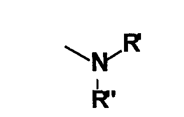 dans lequel R' et R", identiques ou différents, représentent un atome d'hydrogène, un radical alkyle inférieur, un radical mono ou polyhydroxyalkyle, un radical aryle éventuellement substitué ou un reste d'aminoacide ou de peptide ou de sucre ou encore, pris ensemble, forment un hétérocycle,
- R₈ représente un atome d'hydrogène, un radical alkyle linéaire ou ramifié ayant de 1 à 20 atomes de carbone, un radical alkényle, un radical mono ou polyhydroxyalkyle, un radical aryle ou aralkyle éventuellement substitué ou un reste de sucre ou un reste d'aminoacide ou de peptide,
- Y et Z ne peuvent représenter en même temps un atome d'oxygène ou un radical -S(O)ₜ-.
ou un sel ou analogue chiral d'un tel composé, caractérisé en ce que ledit procédé comprend l'étape consistant à mélanger ledit composé bicyclique aromatique à au moins un filtre solaire lipophile.
